**Europäisches Patentamt**

**European Patent Office**

**Office européen des brevets**

(19)

(11) Veröffentlichungsnummer: **0 180 934 B1**

(12) **EUROPÄISCHE PATENTSCHRIFT**

(45) Veröffentlichungstag der Patentschrift: **24.10.90**

(21) Anmeldenummer: **85113948.5**

(22) Anmeldetag: **02.11.85**

(51) Int. Cl.⁵: **B 01 J 20/28,** B 01 J 20/10, B 01 D 15/08

(54) **Verwendung grob gekörnter Schichtsilikate als Adsorbentien für Proteine.**

(30) Priorität: **06.11.84 DE 3440444**

(43) Veröffentlichungstag der Anmeldung:
**14.05.86 Patentblatt 86/20**

(45) Bekanntmachung des Hinweises auf die Patenterteilung:
**24.10.90 Patentblatt 90/43**

(84) Benannte Vertragsstaaten:
**AT BE CH DE FR GB IT LI LU NL SE**

(56) Entgegenhaltungen:
**EP-A-0 058 497**
**DE-A-2 727 143**
**BIOTECHNOLOGY AND BIOENGINEERING, Band 21, 1979, Seiten 1697-1709, John Wiley & Sons, Inc.; T. TOSA et al.: "Immobilization of enzymes and microbial cells using carrageenan as matrix"**
**BIOTECHNOLOGY LETTERS, Band 3, Nr. 1, 1981, Seiten 9-14; K.-D. VORLOP et al.: "Formation of spherical chitosan biocatalysts by ionotropic gelation"**

(73) Patentinhaber: **DECHEMA Deutsche Gesellschaft für Chemisches Apparatewesen, Chemische Technik und Biotechnologie e.V.**
**Theodor-Heuss-Allee 25**
**D-6000 Frankfurt am Main 97 (DE)**

(72) Erfinder: **Dauner, Claudia, Dipl.-Chem.**
**Herzogstrasse 38**
**D-6000 Frankfurt 71 (DE)**
Erfinder: **Borchert, Axel, Dr. Dipl.-Chem.**
**Eppsteiner Strasse 2**
**D-6000 Frankfurt 1 (DE)**
Erfinder: **Buchholz, Klaus, Priv. Doc. Dr. Dipl.-Chem.**
**Rüningerstrasse 44**
**D-3300 Braunschweig (DE)**

(74) Vertreter: **Beil, Hans Christoph, Dr. et al**
**Beil, Wolff und Beil Rechtsanwälte**
**Adelonstrasse 58**
**D-6230 Frankfurt am Main 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft die Verwendung grob gekörnter Schichtsilikate als Adsorbentien für Proteine, beispielsweise bei der Abtrennung von Proteinen aus Rohlösung oder bei der Reinigung und Aufkonzentrierung von Proteinen.

Die Verwendung glimmerartiger Schichtsilikate zur Adsorption von Proteinen ist beispielsweise aus Soil Science, Bd. 48 (1939), S. 467 bis 471 und Proc. 9th Nat. Clay Conf. (1962), S. 520 bis 529 bekannt. Dabei wurde beispielsweise für die Adsorption von Gelatine oder Albumin an Montrillonit eine hohe Adsorptionskapazität bis zu 3 bis 4 g Protein g Schichtsilikat gefunden. Weiterhin ist aus der DE—OS 22 17 745 die Adsorption von Penicillin-Acylase aus einem Zellrohextrakt an Bentonit, einem Schichtsilikat vom Montmorillonit-Typ, zwecks Reinigung bekannt. Aus der DE—OS 26 04 759 ist ein Verfahren zur Reinigung von Gammaglobulin durch Adsorption an glimmerartigen Schichtsilikaten, wie Bentonit, bekannt. Die bisher für diese bekannten Adsorptionen verwendeten Schichtsilikate weisen zwar günstige Adsorptionseigenschaften, d.h. eine hohe Adsorptionskapazität, hohe Selektivität und quantitative Desorbierbarkeit der gewünschten Komponente in aktiver Form, auf, haben jedoch den Nachteil, daß sie nach einmaligem Gebrauch verworfen werden, weil ihre vollständige Abtrennung aus Lösungen aufgrund ihrer feindispersen Struktur mit einem erheblichen technischen Aufwand verbunden ist, der für technische Verfahren nicht geeignet ist. Bisherige Granulierungsverfahren mit diesen Schichtsilikaten führten zu einer erheblichen Abnahme der Adsorptionskapazität und Selektivität der Schichtsilikate.

Andererseits sind aus Biotechnol. Lett., Bd. 3 (1981), Nr. 1, Seite 9 bis 14; 6th Intern. Ferment. Sympos., London (Canada) 1980, Papier F 12.1.12, S. 122 sowie Biotech.Bioeng., Bd. 21 (1779), S. 1697 bis 1707 Verfahren zur Immobilisierung von Enzymen und Einschluß von Zellen in eine Polymermatrix bekannt, bei denen als Polymere beispielsweise Polykationen oder Polyanionen verwendet werden, deren rasche Quervernetzung durch Eintropfen in eine Gegenionen enthaltende Elektrolytlösung erfolgt. Als Beispiele für derartige Polymere werden Chitosan, Alginate, Carrageenan, Carboxymethylcellulose und andere Cellulosederivate sowie ein Copolymerisat aus Styrol und Maleinsäure genannt. Als weitere geeignete Polymere für derartige Einschlußverfahren werden z.B. Agar und Collagen, deren Quervernetzung durch Temperaturänderung erfolgen kann, oder Epoxidharze, die durch Polykondensation vernetzt werden können, genannt. Bei diesen bekannten Verfahren entstehen irreversibel eingeschlossene Biokatalysatoren. Es war daher nicht zu erwarten, daß man bei Anwendung solcher Einschlußverfahren auf Schichtsilikate Produkte erhalten könnte, bei denen die reversible Adsorptionsfähigkeit und Desorbierbarkeit der Schichtsilikate in ausreichendem Maße erhalten bliebe.

Die DE—A—2 727 143 beschreibt ein Verfahren zur Herstellung eines porösen Materials, bei dem eine Aufschlämmung eines sorptiven anorganischen Materials, z.B. Bentonit, in einer Lösung eines in eine unlösliche Form umwandelbaren Zusatzstoffes, z.B. einer wässrigen Lösung eines löslichen Alginats, zu Tröpfchen geformt und mit einem Reagenz in Verbindung gebracht wird, das den Zusatzstoff ausfällt, und die erhaltenen kugelförmigen Teilchen aus dem anorganischen Material und dem Zusatzstoff derart behandelt werden, dass der unlösliche Zusatzstoff zumindest teilweise aus den Teilchen entfernt wird und ein poröses Material ehalten wird. Die letztgenannte Behandlung kann z.B. in einem "Ausbrennen" bestehen. Das auf diese Weise hergestellte Material kann für chromatographische Trennung, ferner als Trägermaterial, auf dem biologisch aktive Substanzen, wie z.B. Enzyme, immobilisiert werden, verwendet, werden.

Der Erfindung liegt die Aufgabe zugrunde, solche grob gekörnten Schichtsilikate zu verwenden, bei denen die vorteilhaften Adsorptions- und Desorptionseigenschaften der Schichtsilikate weitgehend erhalten bleiben, die jedoch gut aus der Adsorptionslösung abtrennbar und wiederverwendbar sind und auch in technischen Apparaten wiederholt und/oder kontinuierlich eingesetzt werden können.

Diese Aufgabe wird erfindungsgemäß dadurch gelöst, daß man Schichtsilikate verwendet, die hergestellt werden, indem man eine Suspension eines Schichtsilikates in einer wäßrigen Polymerlösung herstellt, die erhaltene Suspension in eine Elektrolytlösung eintropft und die entstandenen Körner oder Kugeln gewinnt.

Die nach diesem Verfahren erhaltenen grob gekörnten Schichtsilikate sind in einer Polymermatrix eingeschlossen und weisen eine granuläre oder kugelförmige Gestalt auf, die für ihre Verwendung als Adsorbens überraschenderweise besonders geeignet ist. Sie sind daher gut und praktisch vollständig aus der Adsorptionslösung abtrennbar und wiederverwendbar und können in technischen Apparaten, insbesondere Festbetten, Fließbetten, Rundschüttlern und Rührkesseln, wiederholt und/oder kontinuierlich eingesetzt werden. Durch die große Dichtedifferenz zur flüssigen Phase erfolgt ihre rasche Sedimentation, wodurch auch ein Einsatz in Säulenverfahren möglich ist. Überraschenderweise bleiben im erfindungsgemäßen Verfahren die günstigen Adsorptionseigenschaften der Schichtsilikate, d.h. ihre hohe Adsorptionskapazität und Selektivität und die quantitative Desorbierbarkeit der gewünschten Komponente in aktiver Form, die ihren Einsatz zur Reinigung und Aufkonzentrierung von Proteinen begünstigen, weitgehend erhalten.

Bevorzugt verwendet werden solche Silikate, die hergestellt werden, indem man eine wäßrige Suspension, die 10 bis 60 g eines Schichtsilikats und 7 bis 60 g wasserlösliche, fällbare Polymere pro 1 enthält, bereitet, wobei das Mengenverhältnis von Schichtsilikat zu Polymer, bezogen auf das

2

Trockengewicht, 0,2:1 bis 8:1, vorzugsweise 0,2:1 bis 5:1, beträgt. Dabei erfolgt eine gleichmäßige Verteilung der Suspension des Schichtsilikats in der Polymerlösung und man erhält eine viskose Suspension. Aufgrund der Stabilitätseigenschaften des fertigen Adsorbens ist es nicht ratsam, den Schichtsilikatanteil auf mehr als das 8-fache des Polymeranteils zu steigern. Aus dem gleichen Grund sollte die Polymerkonzentration in der Eintropfsuspension 7 g/l nicht unterschreiten. Diese Suspension wird in ein Eintropfgefäß überführt und von dort durch eine Känule in ein Elektrolytlösung eingetropft. Vorzugsweise werden ein zylindrisches Eintropfgefäß und eine Kanüle mit einem Innendurchmesser von 0,2 bis 0,9 mm verwendet. Das Eintropfen erfolgt vorzugsweise unter Druck. Durch Wahl des Kanülendurchmessers und des aufgebrachten Druckes kann die Partikelgröße gesteuert werden.

Als Schichtsilikate können sowohl glimmerartige Schichtsilikate, wie z.B. Glimmer, Illite, Montmorine, Montmorillonoide, Montmorillonite, Vermiculit oder Batavit, als auch Schichtsilikate vom Strukturtyp des Kaolinits, wie z.B. Kaolin oder Serpentin, eingesetzt werden. Vorzugsweise werden Montmorillonite, wie z.B. Bentonit, verwendet.

Als Polymere können organische wasserlösliche, fällbare Polymere verwendet werden. Beispiele für brauchbare Polymere sind natürliche Polymere, wie Chitosan, Alginate, Carrageenan, Pektinate, Cellulosederivate, Agar oder Agarose, oder synthetische Polymere, wie Epoxidharze. Vorzugsweise werden als Polymere Polykationen oder Polyanionen verwendet, wobei dann die Suspension des Schichtsilikats in der Polymerlösung in eine Gegenionen enthaltende Elektrolytlösung eingetropft wird.

Gegebenenfalls können die nach dem Eintropfen gewonnenen Körner oder Kugeln durch Veränderung des pH-Wertes und/oder der Elektrolytlösung und/oder Verringerung der Temperatur nachgehärtet werden.

Das Schichtsilikat wird als solches mit einer Polymerlösung vermischt oder zuvor in Wasser oder einer Elektrolytlösung suspendiert. Als derartige Elektrolytlösung kann je nach dem verwendeten Polymer beispielsweise für Carrageenan eine Natriumphosphatpufferlösung, für Alginate eine NaCl-Lösung oder für Chitosan eine $CaCl_2$-Lösung verwendet werden. Vorzugsweise wird die Schichtsilikatsuspension zwecks Stabilisierung gegen mikrobiellen Befall mit $NaN_3$ versetzt, beispielsweise in einer Konzentration von $10^{-3}$ mol/l.

Nach einer besonders bevorzugen Ausführungsform der Erfindung wird ein Silikat verwendet, welches als Polymer Chitosan enthält.

Zur Herstellung der Chitosanlösung werden 1—5% Chitosan in destilliertem Wasser aufsuspendiert und nach der Sterilisation im Autoklaven so viel Essigsäure zugesetzt, daß das Chitosan gerade gelöst ist. Diese ist bei einem pH-Wert von ≦5 der Chitosanlösung der Fall. Diese Lösung wird mit einer Schichtsilikatsuspension, z.B. einer Bentonitsuspension, intensiv vermischt. Die resultierenden Konzentrationen betragen für Bentonit 11—50 g/l, für Chitosan 0,7—2%. Die daraus resultierenden Mengenverhältnisse Bentonit:Chitosan betragen zwischen 0,6:1 und 5:1. Man erhalt viskose Suspensionen, die in ein zylindrisches Eintropfgefäß überführt und mit Druck durch eine Kanüle (Innendurchmesser 0,2 bis 0,9 mm) in eine vorzugsweise 1,5%ige Natriumtripolyphosphatlösung vom pH-Wert, 5,5 eingetropft werden. Nach etwa einer Stunde eine die Kugeln zum Schrumpfen in ein Natriumtripolyphosphatlösung vom pH-Wert 8 überführt.

Nach einer anderen bevorzugten Ausführungsform der Erfindung wird als Polymer Carrageenan verwendet. Dabei werden 3—6% Carrageenan in destilliertem $H_2O$ suspendiert und erwärmt, wodurch das Carrageenan in Lösung geht. Nach erfolgter Zugabe der Schichtsilikatsuspension, z.B. einer Bentonitsuspension, wobei die Konzentration in der Eintropfsuspension für Carrageenan 1 bis 3% und für Bentonit 10 bis 50 g/l betragen sollten, ist darauf zu achten, daß die Gesamtsuspension auf der richtigen Temperatur gehalten wird, da bei niedriger Temperatur einige Carrageenanpräparate zu gelieren beginnen. Daher wird auch das Eintropfgefäß thermostatisiert. Das Mengenverhältnis von Schichtsilikat zu Carrageenan kann im Bereich von 0,2:1 bis 5:1 variiert werden. Im übrigen läuft das Verfahren analog der Herstellung des Chitosanproduktes ab. Als Vernetzerlösung wird vorzugsweise eine 2%ige $CaCl_2$-Lösung eingesetzt, und zur weiteren Aushärtung wird in eine 2%ige KCl-Lösung überführt.

Wenn ein Alginat als Polymer verwendet wird, sollten die Konzentrationen in der Eintropfsuspension für das Alginat 2 bis 6% und für das Schichtsilikat 10 bis 40 g/l betragen. In diesem Fall ist eine Erwärmung und Thermostatisierung der Polymerlösung bzw. der Eintropfsuspension nicht erforderlich. Als Vernetzungslösung (Elektrolytlösung) kann eine vorzugsweise 2%ige $CaCl_2$-Lösung verwendet werden.

Nach einer weiteren bevorzugten Ausführungsform kann als Polymer eine Gemisch aus einem Polykation oder Polyanion mit einem Epoxidharz und entsprechendem Härter verwendet werden. In diesem wird eine solche Konzentration an Polykation oder Polyanion verwendet, daß beim Eintropfen der Suspension des Schichtsilikates in der Polymerlösung in eine die entsprechenden Gegenionen enthaltende Elektrolytlösung Körner gebildet werden. Nach Aushärten des Epoxidharzes wird das Polykation oder Polyanion mit Wasser oder einer geeigneten Elektrolytlösung aus den Körnern herausgelöst. Die Konzentration an Polykation oder Polyanion muß auch so gewählt werden, daß nach ihrem Herauslösen aus den Körnern eine ausreichende Porosität des Adsorbens gewährleistet ist.

Die wie vorstehend dargelegt hergestellten grob gekörnten Schichtsilikate, die aus in einer Polymermatrix eingeschlossenen Schichtsilikatenen bestehen, werden erfindungsgemäß als Adsorbentien für Proteine, beispielsweise zur Reinigung von Proteinen, verwendet. Ein Beispiel für ein solches Protein, das erfindungsgemäß aufgereinigt werden kann, ist das Enzym Penicillin-Acylase (PA). PA ist ein hydrolytisch wirkendes Enzym, das zur Herstellung von 6-Aminopenicillansäure (6—APA) im industriellen Maßstab eingesetzt wird. 6—APA ist ein Edukt bei der Herstellung semisynthetischer Antibiotika. Der isoelektrische Punkt von PA liegt bei einem pH-Wert von 6,4 bis 6,8, so daß bei einem niedrigeren pH-Wert, vorzugsweise von bis 5, eine positive Gesamtladung vorliegt, die mit der negativen Schichtladung der Silikate in Wechselwirkung tritt und so das Enzym in der Hauptsache durch elektrostatisches Wechselwirkung adsorptiv gebunden wird. Desorption kann durch Erhöhung des pH-Wertes auf einen Wert oberhalt 7 erreicht werden, vorzugsweise auf einen pH-Wert im Bereich von 7 bis 8.

Die folgenden Beispiele dienen zur weiteren Erläuterung der Erfindung.

Beispiel 1

1,5 g α-Carrageenan (α-Carrageenan Genugel X—0828 von Kobenhavens Pektinfabrik, Dänemark) wurden in 25 ml destilliertem $H_2O$ suspendiert und auf 45°C erwärmt, bis das Carrageenan sich vollständig aufgelöst hatte. Anschließende wurden unter Rühren 25 ml Bentonitsuspension (60 mg/ml in $5 \cdot 10^{-3}$ mol/l Na-Phosphatpuffer pH 5) zugegeben. Die resultierenden Konzentrationen betrugen sowohl für Carrageenan als auch Bentonit 30 mg/ml. Die Gesamtsuspension wurde aus einem auf 45°C thermostatisierten Eintropfgefäßt unter Druck durch eine Kanüle (Innendurchmesser 0,7 mm) in 2%ige $CaCl_2$-Lösung eingetropft. Durch einen äußeren Zylinder parallel zur Kanüle wurde Druckluft zugeführt, die die am Ausgang der Kanüle gebildeten Tropfen zum Abreißen brachte. Abschließend wurden die Kugeln in 2%ige KCl-Lösung überführt und zum Aushärten über Nacht bei 4°C aufbewahrt.

Beispiel 2

Die Arbeitsweise von Beispiel 1 wurde mit der Abweichung wiederholt, daß an Stelle einer Bentonitsuspension der Konzentration 60 mg/ml eine Suspension der Konzentration 24 mg/ml verwendet wurde.

Biespiel 3

1 g Chitosan in 50 ml $H_2O$ wurde im Autoklaven sterilisiert, und anschließend wurde das Chitosan durch Zugabe von 50 µl $CH_3COOH$ gelöst. 17,5 ml dieser Lösung wurden mit 29,17 ml Bentonitsuspension (60 mg/ml in $CaCl_2$ $5 \cdot 10^{-3}$ mol/l, pH 5) und 3,3 ml $CaCl_2$ $5 \cdot 10^{-3}$ mol/l zusammengegeben und so lange gerührt, bis eine homogene Verteilung erreicht war. Das Gesamtvolumen betrug 50 ml, mit einer Bentonitkonzentration von 35 mg/ml und Chitosankonzentration von 7 mg/ml, das Mengenverhältnis bezogen auf Trockengewicht zwischen Bentonit und Chitosan betrug 5:1. Diese Suspension wurde entsprechende der Arbeitsweise von Beispiel 1 mit der Abweichung, daß die Thermostatisierung des Eintropfgefäßes entfiel, in Na-tripolyphosphatlösung 1,5% pH 5 eingetropft, nach etwa 1 h in Na-tripolyphosphat, 1,5%, pH 8 überführt und darin über Nacht gerührt, um eine Schrumpfung bzw. Nachhärtung der Partikel zu erreichen.

Beispiel 4

Die Arbeitsweise von Beispiel 3 wurde mit der Abweichung wiederholt, daß 23,5 ml Chitosanlösung, 15,67 ml Bentonitsuspension und 10,83 ml $CaCl_3$-Lösung zusammengegeben wurden, so daß die resultierenden Konzentrationen 18,8 mg/ml Bentonit und 9,4 mg/ml Chitosan in der Eintropfsuspension betrugen. Das Mengenverhältnis, bezogen auf Trockengewicht, von Bentonit zu Chitosan betrug 2:1.

Beispiel 5

100 mg Bentonit in 1,2 ml NaCl-Lösung $5 \cdot 10^{-3}$ mol/l wurden mit 0,4 ml eines Epoxidharzes ("Epikote DX—255" der Deutsche Shell Gesellschaft) vermischt Anschließend wurden 0,8 ml einer 25%igen wäßrigen Lösung einer Polyaminoamid-Härterkomponente ("Epilink 360" der Akzo Chemie, Düren) (0,4 ml Härter-Lösung und 0,4 ml destilliertes $H_2O$) zugegeben und durch Rühren gut vermischt, wodurch die Polykondensation des Epoxidharzes eingeleitet wurde. Diese Suspension wurde mit 1,2 ml einer 8%igen Na-Alginatlösung (Manucol LB, Alginate Industries GmbH, Hamburg) gut vermischt und anschließend nach der Arbeitsweise von Beispiel 3 durch eine Kanüle mit einem Innendurchmesser von 0,4 mm in eine 2%ige $CaCl_2$-Lösung eingetropft. Man rührte noch etwa eine Stunde und trocknete anschließend 24 Stunden an der Luft.

Die trockenen, bezüglich des Epoxidharzes ausgehärteten Kugeln wurden nun in 0,1 mol/l Phosphatpuffer pH 7 gerührt. Hierbei wurde das Alginat als Polyelektrolyt aus den Kugeln herausgelöst und man erhielt ein poröses Adsorbens.

Die in den vorstehenden Beispielen hergestellten Präparate wurden zum einen hinsichtlich ihrer Adsorptionskapazität, Desorbierbarkeit im Vergleich zu dem unbehandelten Schichtsilikat sowie ihrer mehrfachen Verwendbarkeit, andererseits bezüglich ihrer Stabilität beim Einsatz in technischen Apparaturen, z.B. im Fließbett, Rührkessel und Rundschüttler, getestet.

Adsorptionsmessungen wurden durchgeführt mit Penicillin-Acylase (PA). Die Konzentrationsangabe erfolgt in Enzymeinheiten E; eine Enzymeinheit E ist die Enzymmenge, die 1 µ mol Substrat/min umsetzt. Das hier verwendete Substrat war Benzylpenicillin, die Reaktion wurde bei pH 8 und 25°C mittels automatischer Titration verfolgt.

Die Kapazitäten der erfindungsgemäß hergestellten Präparate sind in der nachstehenden Tabelle I

4

zusammengestellt. Sie unterscheiden sich deutlich, je nachdem, welche Polymermatrix zum Einschluß des Schichtsilikats verwendet wurde. Unbehandeltes Bentonit besaß eine Kapazität für PA von 8000 E/g, die mit Carrageenan hergestellten Präparate eine solche von 7000 bis 12 000 E/g. Mit Chitosan hergestellte Präparate zeigten ein Adsorptionsvermögen zwischen 1000 und 2000 E/g; bezogen auf den jeweiligen Bentonitanteil im Präparat, der die 2 bis 5fache Menge der Polymermenge betrug, lagen die Adsorptionswerte entsprechend höher. Dies bedeutet, daß durch den Einschluß in Chitosan die Kapazität des Bentonit auf 1/3 des ursprünglichen Wertes vermindert wurde, sie lag jedoch weiterhin beträchtlich über der anderer Adsorbentien. So können mit einem im Handel erhältlichen, auf übliche Weise granulierten Bentonitpräparat (Harwell, U.K.) nur 45 E/g Präparat adsorbiert werden. Selbst das Epoxidharz-Präparat, dessen Kapazität mit 72 E/g gering war, lag fast um den Faktor 2 darüber. Dessen geringe Kapazität wurde vermutlich auch durch den geringen Bentonitgehalt des Präparates verursacht; bezieht man die Adsorptionswerte auf den Bentonitanteil so lag auch das Adsorptionsvermögen des Epoxid-Präparates mit 640 E/g Bentonit um eine Zehnerpotenz über dem des bekannten granulierten Produktes. Auch im Vergleich zu anderen, zur Adsorption von PA befähigten Stoffen, z.B. einem Präparat aus porösen Glasteilchen (Controlled Pore Glass, d.h. Glas mit eng eingestellter Porenweite) das 260 E/g adsorbieren kann, zeigten die eingeschlossenen Bentonitpräparate deutliche bessere Adsorptionsfähigkeit.

## Tabelle I

### Adsorptionskapazität verschiedener Präparate bei der Adsorption von Penicillin-Acylase

| Präparat | Kapazität E/g Präparat |
|---|---|
| nativer Bentonit (Vergleich) | 8000 |
| Bentonit/Carrageenan 1:1 Produkt des Beispiels 1 | 7000 |
| Bentonit/Carrageenan 1:5 Produkt des Beispiels 2 | 12000 |
| Bentonit/Chitosan 5:1 Produkt des Beispiels 3 | 2000 |
| Bentonit/Chitosan 2:1 Produkt des Beispiels 4 | 1190 |
| Bentonit/Epoxid 1:8 Produkt des Beispiels 5 | 72 |
| granuliertes Bentonit-Präparat (Vergleich) | 45 |

Die Desorption der PA erfolgte von nativem Bentonit quantitativ in aktiven Zustand, im Gegensatz zu Fremdprotein, da nur 60—70% des absorbierten Gesamtproteins desorbiert wurden. Ebenfalls quantitativ erfolgte die Desorption der aktiven Komponente von mit Carrageenan hergestellten Präparaten, während Fremdprotein im ersten Desorptionsschritt nur zur 60—80% (Carrageenan/Bentonit 5:1) bzw. 40—70% (Carrageenan/Bentonit 1:1) erfolgte, je nach Beladung des Präparates. Je höher das Präparat beladen war, desto größer war die Proteinmenge, die desorbiert wurde. Von Chitosanträgern wurden nach 24 h Adsorptionszeit zwischen 70—100% des adsorbierten aktiven Enzyms desorbiert, bei niedrigen Beladungen sogar nur soch 50%. Erfolgte die Adsorption nur über 6 h, so erfolgte die Desorption nahezu quantitativ. Der Anteil des desorbierten Gesamtproteins betrug hier 30—45%.

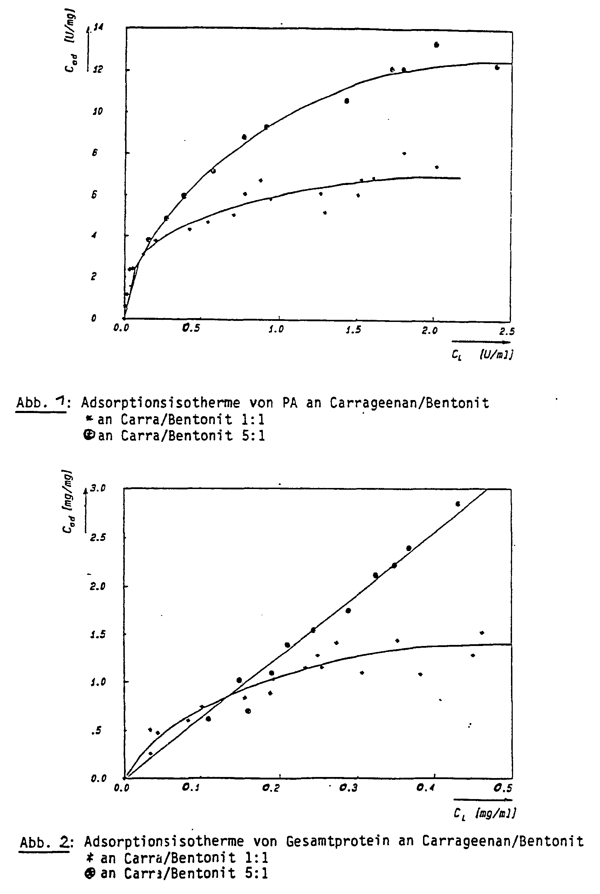

Sowohl im Adsorptions- als auch im Desorptionsschritt erfolgte eine Anreicherung der gewünschten Komponente, da diese einerseits bevorzugt aus der Lösung eines Gemisches adsorbiert wurde, bei pH-Verschiebung jedoch auch bevorzugt wieder desorbiert wurde. Die bevorzugte Adsorption läßt sich sowohl durch eine Abnahme der spezifischen Aktivität im Adsorptionsüberstand als auch durch den deutlich steileren Anstieg der Isotherme für die Enzymadsorption im Gegensatz zur Isotherme für die Adsorption von Gesamtprotein belegen. Diese Adsorptionisothermen sind in den Abb. 1 und 2 wiedergegeben.

Die Abnahme der spezifischen Aktivität im Adsorptionsüberstand ist in der folgenden Tabelle II zusammengestellt. Daraus ist ersichtlich, daß die Selektivität zugunsten der gewünschten Komponente mit abnehmender Restkonzentration im Adsorptionsüberstand größer wurde. Sie war besonders stark ausgeprägt bei niedrigen Restkonzentrationen in Lösung, wie sie bei der Gewinnung von Proteinen angestrebt werden.

EP 0 180 934 B1

## TABELLE II

Kapazität und Selektivität bei der Adsorption von PA an verschiedenen Präparaten bei verschiedenen Restkonzentrationen im Adsorptionsüberstand

| Präparat | spez.Akt. eingesetzte Enzymlsg. · E/mg Protein | bei Restkonzentration im Adsorptionsüberstand von | | | | | |
|---|---|---|---|---|---|---|---|
| | | 0,25 E/ml | | 0,75 E/ml | | 1 E/ml | |
| | | Kapazität | spez.Akt. im Adsor. Überstand | Kapazität | spez.Akt. im Adsor. Überstand | Kapazität | spez. Akt. im Adsor. Überstand |
| | | E/g Adsorb. | E/mg P. | E/g A. | E/mg P. | E/g A. | E/mg P. |
| nativer Bentonit | 6,1 | 8500 | 2 | 8500 | 4,75 | 8000 | 5,75 |
| Bentonit/ Chitosan 5:1 | 7,5 | 1800 | 5 | 2300 | 7,5 | 2400 | 7,5 |
| Bentonit/ Carrageenan 1:1 | 4,5 | 4000 | 2 | 5500 | 3,5 | 6000 | 3,9 |
| Bentonit/ Carrageenan 1:5 | 5,3 | 5000 | 2,1 | 9200 | 4 | 10500 | 4,5 |

Insgesamt kann durch einen Ad- und Desorptionsschritt eine Anreicherung der aktiven Komponente um den Faktor 2 erreicht werden, sowohl bei nativem Bentonit als auch bei den erfindungsgemäß hergestellten Präparaten.

Bei einem Einsatz der Präparate über mehrere Ad- und Desorptionszyklen erfolgte zwar eine Abnahme der Adsorptionskapazität. Die verbleibende Kapazität bewegte sich jedoch noch in einer akzeptablen Größenordnung. So betrug der Kapazitätsschwund sowohl bei Carrageenanals auch Chitosanpräparten im Verlauf von vier Ad-/Desorptionszyklen 25% für die Adsorption von aktivem Enzym.

Im Rundschüttler waren alle Präparate bei einer Umdrehungszahl von 250 U/min und T = 25°C über einen Zeitraum von mindestens 16 Tagen stabil in unterschiedlichen Medien in einem pH-Bereich von 5—8 und Salzkonzentration zwischen $5 \cdot 10^{-3}$ mol/l und 1 mol/l. Wichtig ist jedoch die Anwesenheit von die Polymermatrix quervernetzenden Ionen in geringen Konzentrationen (z.B., KCl 0,2% für Carrageenan, Na-tripolyphosphat $10^{-3}$ mol/l für Chitosan). Die Grenzen in der pH-Breite sind jedoch nicht durch die Stabilitätsgrenzen der verschiedenen Polymermatrices gegeben, sondern dadurch, daß das für die Adsorptionsuntersuchungen verwendete Enzym PA nur von pH 5 bis 8 stabil ist. Das kommerziell erhältliche granulierte Bentonitpräparat (Harwell) zeigte unter den angegebenen Bedingungen leichten Abrieb, feststellbar durch eine Trübung der Lösung.

Im Fließbett (Säulendurchmesser 4 cm, T = 25°C) waren die getesteten Präparatef stabil bis zu Strömungsgeschwindigkeiten, die zum Austragen der Partikel führten.

Entsprechend ihrer Größe (0,2—1,5 mm Durchmesser) waren dies Strömungsgeschwindigkeiten von 70—150 ml/min ≙22,5—50 cm/min Lineargeschwindigkeit für das Präparat Carrageenan/Bentonit 5:1, von 100—350 ml/min entsprechend einer Lineargeschwindigkeit von 31—110 cm/min für das Präparat Chitosan/Bentonit 1:5. Das Testmedium entsprach dem bei der Adsorption eingesetzten (pH 5, KCl 0,2%/ $KH_2PO_4$ $10^{-3}$ mol/l für Carrageenan, pH 5, Na-tripolyphosphat 0,05% für Chitosan), die Testzeit betrug mindestens 70 h.

Auch im Festbett (Säulendurchmesser 5,7 mm) erwies sich das Chitosanpräparat (Chit/Bent. 1,4:1) bei einer Fließgeschwindigkeit von 0,78 ml/min ≙3,06 cm/min und einem pH-Wert von 8 über 400 h stabil.

**Patentansprüche**

1. Verwendung grob gekörnter Schichtsilikate als Adsorbentien für Proteine, hergestellt durch Eintropfen einer Suspension eines Schichtsilikates in eine wäßrige Polymerlösung, Eintropfen der so erhaltenen Suspension in ein Elektrolytlösung und Gewinnung der entstandenen Körner oder Kugeln.

2. Verwendung nach Anspruch 1, dadurch gekennzeichnet, daß die Schichtsilikate hergestellt sind aus einer wäßrigen Suspension, die 10 bis 60 g eines Schichtsilikates und 7 bis 60 g wasserlösliche, fällbare Polymere pro 1 enthält, wobei das Mengenverhältnis von Schichtsilikat zu Polymer, bezogen auf Trockengewicht, 0,2:1 bis 8:1 beträgt, und wobei die erhaltene Suspension durch eine Kanüle in die Elektrolytlösung eingetropft wird.

3. Verwendung nach Anspruch 1 oder 2, dadurch gekennzeichnet, daß die Schichtsilikate durch Eintropfen in eine Polymerlösung aus Polykationen oder Polyanionen hergestellt sind.

4. Verwendung nach einem der Ansprüche 1 bis 3, dadurch gekennzeichnet, daß die Schichtsilikate hergestellt sind, indem die entstandenen Körner oder Kugeln durch Veränderung des pH-Wertes und/oder der Elektrolytlösung und/oder Verringerung der Temperatur nachgehärtet werden.

5. Verwendung nach einem der Ansprüche 1 bis 4, dadurch gekennzeichnet, daß die Schichtsilikate hergestellt sind aus einem glimmerartigen Schichtsilikat, insbesondere einem Montmorillonit.

6. Verwendung nach einem der Ansprüche 1 bis 5, dadurch gekennzeichnet, daß die Schichtsilikate hergestellt sind unter Verwendung von Chitosan als Polymer, einer 0,5—2%igen Natriumtripolyphosphatlösung und einem pH-Wert von 5,5 als Elektrolytlösung und einer 0,5—2%igen Natriumtripolyphosphatlösung und einem pH-Wert von 8,0 zur Nachhärtung.

7. Verwendung nach einem der Ansprüche 1 bis 5, dadurch gekennzeichnet, daß das Schichtsilikat als Polymer Carrageenan aufweist und die Temperatur der Carrageenanlösung und der Suspension des Schichtsilikats bei dessen Herstellung oberhalb des Gelierpunktes liegt, die verwendete Elektrolytlösung eine 2%ige $CaCl_2$- oder KCl-Lösung ist und das Schichtsilikat bei verringerter Temperatur und/oder Wechsel der Elektrolytlösung nachgehärtet ist.

8. Verwendung nach einem der Ansprüche 1 bis 5, dadurch gekennzeichnet, daß das Schichtsilikat als Polymer ein Alginat, insbesondere Natriumalginat, aufweist und als Elektrolytlösung eine 2%ige $CaCl_2$-Lösung verwendet wird.

9. Verwendung nach einem der Ansprüche 1 bis 5, dadurch gekennzeichnet, daß man ein Schichtsilikat verwendet, das als Polymer ein Gemisch aus einem Polykation oder Polyanion mit einem Epoxidharz und entsprechendem Härter aufweist und wobei die Suspension des Schichtsilikates in der Polymerlösung in eine die entsprechenden Gegenionen enthaltende Elektrolytlösung eingetropft wird und nach Aushärten des Epoxidharzes das Polykation oder Polyanion mit Wasser oder einer geeigneten Elektrolytlösung aus den Körnern herausgelöst wird.

# EP 0 180 934 B1

## Revendications

1. Application comme adsorbants pour protéines de silicates lamellaires en gros grains, préparés par introduction goutte à goutte d'une suspension d'un silicate lamellaire dans une solution aqueuse de polymère, introduction goutte à goutte de la suspension ainsi obtenue dans une solution d'électrolyte et obtention des grains ou billes apparus.

2. Application selon la revendication 1, caractérisée en ce que les silicates lamellaires sont préparés à partir d'une suspension aqueuse qui contient de 10 à 60 g d'un silicate lamellaire et 7 à 60 g de polymères précipitables solubles dans l'eau par litre, dans laquelle le rapport quantitatif du silicate lamellaire au polymère rapporté au poids sec, s'élève à 0,2:1 à 8:1, et où la suspension obtenue est introduite goutte à goutte par une canule dans la solution d'électrolyte.

3. Application selon la revendication 1 ou 2, caractérisée en ce que les silicates lamellaires sont préparés par introduction goutte à goutte dans une solution de polymère faite de polycations ou de polyanions.

4. Application selon l'une des revendications 1 à 3, caractérisée en ce qu'on prépare les silicates lamellaires en durcissant les grains ou billes apparus par modification du pH et/ou de la solution d'électrolyte et/ou abaissement de la température.

5. Application selon l'une des revendications 1 à 4, caractérisée en ce qu'on prépare les silicates lamellaires à partir d'un silicate lamellaire micacé, en particulier d'une montmorillonite.

6. Application selon l'une des revendications 1 à 5, caractérisée en ce qu'on prépare les silicates lamellaires en utilisant du chitosan comme polymère, une solution de tripolyphosphate de sodium à 0,5—2% ayant un pH de 5,5 comme solution d'électrolyte et une solution de tripolyphosphate de sodium à 0,5—2% ayant un pH de 8,0 pour le durcissement ultérieur.

7. Application selon l'une des revendications 1 à 5, caractérisée en ce que le silicate lamellaire contient comme polymère la carraghénine et en ce que la température de la solution de carraghénine et de la suspension de silicate lamellaire lors de sa préparation se situe au-dessus du point de gélification, en ce que la solution d'électrolyte utilisée est une solution à 2% de $CaCl_2$ ou de KCl, et en ce que le silicate lamellaire est durci ultérieurement en abaissant la température et/ou en changeant la solution d'électrolyte.

8. Application selon l'une des revendications 1 à 5, caractérisée en ce que le silicate lamellaire présente comme polymère un alginate, en particulier l'alginate de sodium, et en ce qu'on utilise comme solution d'électrolyte une solution à 2% de $CaCl_2$.

9. Application selon l'une des revendications 1 à 5, caractérisée en ce qu'on utilise un silicate lamellaire qui présente comme polymère un mélange d'un polycation ou polyanion avec une résine époxyde et un durcissant correspondant et où l'on introduit goutte à goutte la suspension de silicate lamellaire en solution de polymère dans une solution d'électrolyte contenant les ions complémentaires correspondants et en ce qu'après durcissement de la résine époxyde on extrait des grains par dissolution le polycation ou le polyanion avec de l'eau ou une solution d'électrolyte appropriée.

## Claims

1. The use of coarse-grain layer silicates — prepared by dropwise addition of a suspension of a layer silicate to an aqueous polymer solution, dropwise addition of the resulting suspension to an electrolyte solution and recovery of the grains or beads formed — as adsorbents for proteins.

2. The use claimed in claim 1, characterized in that the layer silicates are prepared from an aqueous suspension containing 10 to 60 g of a layer silicate and 7 to 60 g water-soluble precipitatable polymers per 1, the quantitative ratio of layer silicate to polymer, based on dry weight, being 0.2:1 to 8:1 and the suspension obtained being added dropwise to the electrolyte solution through a cannula.

3. The use claimed in claim 1 or 2, characterized in that the layer silicates are prepared by dropwise addition to a polymer solution of polycations or polyanions.

4. The use claimed in any of claims 1 to 3, characterized in that the layer silicates are prepared by post-hardening of the grains or beads formed by changing the pH value and/or the electrolyte solution and/or by lowering the temperature.

5. The use claimed in any of claims 1 to 4, characterized in that the layer silicates are prepared from a mica-like layer silicate, more particularly a montmorillonite.

6. The use claimed in any of claims 1 to 5, characterized in that the layer silicates are prepared using chitosan as polymer, a 0.5—2% sodium tripolyphosphate solution having a pH value of 5.5 as the electrolyte solution and a 0.5—2% sodium tripolyphosphate solution having a pH value of pH of 8.0 for post-hardening.

7. The use claimed in any of claims 1 to 5, characterized in that the layer silicate contains carrageenan as polymer and the temperature of the carrageenan solution and the suspension of the layer silicate during its preparation is above the gelation point, the electrolyte solution used is a 2% $CaCl_2$ or KCl solution and the layer silicate is post-hardened by lowering the temperature and/or changing the electrolyte solution.

8. The use claimed in any of claims 1 to 5, characterized in that the layer silicate contains an alginate, more particularly sodium alginate, as polymer and a 2% $CaCl_2$ solution is used as the electrolyte solution.

9. The use claimed in any of claims 1 to 5, characterized in that the layer silicate used contains a mixture

9

of a polycation or polyanion with an epoxy resin and corresponding catalyst as polymer and the suspension of the layer silicate in the polymer solution is added dropwise to an electrolyte solution containing the corresponding counterions and, after curing of the epoxy resin, the polycation or polyanion is washed out from the grains with water or a suitable electrolyte solution.

Abb. 1: Adsorptionsisotherme von PA an Carrageenan/Bentonit
‍   * an Carra/Bentonit 1:1
‍   ⊕ an Carra/Bentonit 5:1

Abb. 2: Adsorptionsisotherme von Gesamtprotein an Carrageenan/Bentonit
‍   * an Carra/Bentonit 1:1
‍   ⊕ an Carra/Bentonit 5:1